(19) Europäisches Patentamt
European Patent Office
Office européen des brevets

(11) EP 0 508 370 B1

# (12) EUROPÄISCHE PATENTSCHRIFT

(45) Veröffentlichungstag und Bekanntmachung des Hinweises auf die Patenterteilung:
**03.07.1996 Patentblatt 1996/27**

(21) Anmeldenummer: **92105989.5**

(22) Anmeldetag: **07.04.1992**

(51) Int Cl.[6]: **C07D 471/04**, C07D 487/04, C07D 495/04, C07D 243/38, A61K 31/55
// (C07D471/00, 243:00, 221:00), (C07D487/04, 243:00, 209:00), (C07D495/04, 333:00, 243:00)

(54) **Kondensierte Diazepinone, Verfahren zu ihrer Herstellung und diese Verbindungen enthaltende Mittel zur Behandlung von Erkrankungen des Zentralnervensystems und zur Förderung der cerebralen Durchblutung**

Condensed diazepinones, process for their preparation and compositions containing them for the treatment of the central nervous system and to enhance cerebral perfusion

Diazépinones condensées, procédé pour leur préparation et compositions les contenant pour le traitement de maladies du système nerveux central et pour augmenter la perfusion cérébrale

(84) Benannte Vertragsstaaten:
**AT BE CH DE DK ES FR GB GR IT LI LU NL PT SE**

(30) Priorität: **12.04.1991 DE 4112014**

(43) Veröffentlichungstag der Anmeldung:
**14.10.1992 Patentblatt 1992/42**

(73) Patentinhaber: **Dr. Karl Thomae GmbH**
**D-88397 Biberach (DE)**

(72) Erfinder:
- **Eberlein, Wolfgang, Dr.**
**W-7950 Biberach 1 (DE)**
- **Mihm, Gerhard, Dr.**
**W-7950 Biberach 1 (DE)**
- **Engel, Wolfhard, Dr.**
**W-7950 Biberach 1 (DE)**
- **Rudolf, Klaus, Dr.**
**W-7950 Biberach 1 (DE)**
- **Doods, Henri, Dr.**
**W-7951 Warthausen (DE)**
- **Ziegler, Harald, Dr.**
**W-7950 Biberach 1 (DE)**
- **Entzeroth, Michael, Dr.**
**W-7951 Warthausen (DE)**

(56) Entgegenhaltungen:
**EP-A- 0 156 191** **EP-A- 0 312 895**
**EP-A- 0 402 734**

Bemerkungen:
Die Akte enthält technische Angaben, die nach dem Eingang der Anmeldung eingereicht wurden und die nicht in dieser Patentschrift enthalten sind.

EP 0 508 370 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft kondensierte Diazepinone, Verfahren zu ihrer Herstellung und diese Verbindungen enthaltende Mittel zur Behandlung von Erkrankungen des Zentralnervensystems und zur Förderung der cerebralen Durchblutung.

Aus EP-A-0 039 519 und 0 057 428 sowie aus US-A-3.660.380; 3.691.159; 4.213.984; 4.213.985; 4.210.648; 4.410.527; 4.424.225; 4.424.222 und 4.424.226 sind bereits kondensierte Diazepinone mit ulcushemmenden und magensaftsekretionshemmenden Eigenschaften bekannt.

In EP-A-0 156 191 (US-Patent 4.550.107) und EP-A-0 312 895 ist für kondensierte Diazepinone beschrieben, daß durch Einführung von Alkylaminoacyl- oder Dialkylaminoacylresten gegenüber den Verbindungen der obengenannten Publikationen völlig andersartige pharmakologische Eigenschaften, nämlich günstige Effekte auf die Herzfrequenz, induziert werden können. Dies gilt auch für die Verbindungen der EP-A-0 213 293, EP-A-0 254 955, EP-A-0 273 239, EP-A-0 306 698, DE-A-38 00 986, DE-A-38 02 334, DE-A-3 819 444, DE-A-38 20 346 und DE-A-38 20 345. Die Verbindungen dieser Publikationen sind auf Grund ihrer günstigen Effekte auf die Herzfrequenz und angesichts fehlender magensäuresekretionshemmender, salivationshemmender und mydriatischer Einflüsse als vagale Schrittmacher zur Behandlung von Bradycardien und Bradyarrhythmien in der Human- und Veterinärmedizin geeignet. In der EP-A2-0 402 734 werden kondensierte Diazepinone als Mittel zur Behandlung von Erkrankungen des Zentralnervensystems und zur Förderung der cerebralen Durchblutung beschrieben. Diese Mittel eignen sich zur Anwendung in der Geriatrie und bei Migräne. Eine Reihe der dort beschriebenen Verbindungen zeigt eine gute ZNS-Gängigkeit und läßt sich daher zur Therapie von Erkrankungen des Zentralnervensystems, insbesondere des Morbus Alzheimer einsetzen.

Nach den bisher abgeleiteten Struktur-Wirkungs-Beziehungen gilt das Vorhandensein eines terminalen basischen N-Atoms in der Seitenkette als essentiell für muskarinische Antagonisten mit hoher $M_2$-Selektivität (vgl. hierzu US-Patent 4.550.107, EP-A2-0.402.734 und Engel et al. in J. Med. Chem. 32, 1718 (1989)).

Überraschend wurde nun gefunden, daß durch Acylierung des terminalen basischen Stickstoffatoms der an dem Aminostickstoff des Diazepinonringes gebundenen Seitenkette hochaffine $M_2$-selektive muskarinische Antagonisten resultieren.

Die erfindungsgemäßen "Carboxamide" zeigen darüber hinaus im Vergleich zu den bisher beschriebenen $M_2$-selektiven Antagonisten ein wesentlich verbessertes $M_2/M_1$-Verhältnis, das heißt, die neuen Verbindungen weisen ein wesentlich günstigeres Verhältnis der Bindungsaffinitäten gegenüber $M_2$-Rezeptoren im Vergleich zu $M_1$-Rezeptoren auf.

Die erfindungsgemäßen Verbindungen können aufgrund ihrer $M_2$-Selektivität zur Behandlung von Bradykardien und Bradyarrhythmien in der Human- und Veterinärmedizin eingesetzt werden. Angesichts günstiger Effekte auf die cerebrale Durchblutung sind die Verbindungen insbesondere auch zur Anwendung in der Geriatrie und bei Migräne geeignet. Wie sich im Tierversuch (Ratte) zeigte, steigern sie die Lernfähigkeit alter Tiere.

Die erfindungsgemäßen, kondensierten Diazepinone sind zum Teil hochlipophil und erfüllen damit eine wesentliche Voraussetzung für eine gute Gehirngängigkeit. Gleichzeitig führt diese hohe Lipophilie zu einem raschen Abfall des Blutspiegels, so daß eine Beeinflussung der Herzfrequenz unterbleibt. $M_2$-selektive Antagonisten, die eine günstige ZNS-Penetration aufweisen und gleichzeitig eine $M_2/M_1$-Selektivität besitzen, sind besonders zur Therapie von Erkrankungen des Zentralnervensystems, insbesondere des Morbus Alzheimer geeignet.

Bei den erfindungsgemäßen Verbindungen handelt es sich um kondensierte Diazepinone der allgemeinen Formel I

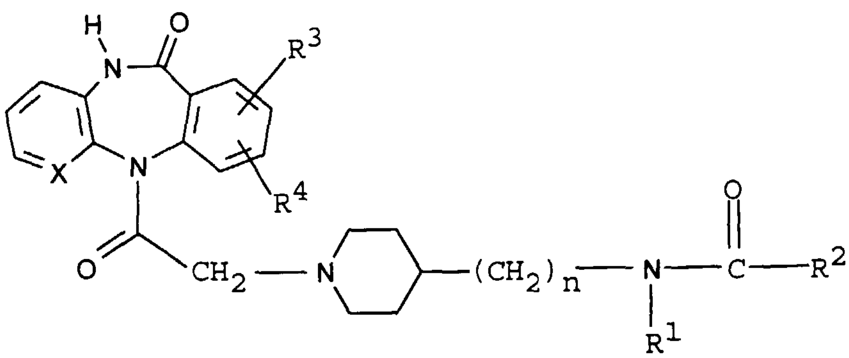

(I),

in der

X ein Stickstoffatom,

n die Zahlen 3 und 4

$R^1$ eine geradkettige Alkylgruppe mit 1 bis 4 Kohlenstoffatomen,

$R^2$ eine verzweigte Alkylgruppe mit 4 bis 6 Kohlenstoffatomen, eine Cyclopropyl-, Cyclobutyl-, Cyclopentyl-, Cyclohexyl-, Cycloheptyl-, Methylcyclopentyl-, Methylcyclohexyl-, Methylcycloheptyl- oder Adamantylgruppe,

$R^3$ und $R^4$ Wasserstoffatome oder einer dieser Reste eine Methylgruppe oder ein Halogenatom bedeuten,

und die Verbindung der Formel I, in der X die =CH-Gruppe,

n die Zahl 3,

$R^1$ die Ethylgruppe,

$R^2$ die 1,1-Dimethylbutylgruppe,

$R^3$ und $R^4$ Wasserstoffatome bedeuten.

Ganz besonders bevorzugt sind hierbei die Verbindungen, in denen $R^1$ eine Ethylgruppe darstellt.

Als verzweigte Alkylgruppen kommen beispielsweise die 1,1-Dimethyl-ethyl-, 1,1-Dimethyl-propyl-, 1,1-Dimethylbutyl-, 1-Ethyl-1-methyl-ethyl-, 1-Ethyl-1-methyl-propyl-, 1-Propyl-1-methyl-ethyl-, 1-Ethyl-ethyl-, 1,1-Diethyl-ethyl oder 1,1-Dimethyl-butylgruppe in Betracht.

Gegenstand der Erfindung sind auch die Säureadditionssalze mit anorganischen oder organischen Säuren, z. B. mit Salzsäure, Bromwasserstoffsäure, Schwefelsäure, Fumarsäure, Maleinsäure, Äpfelsäure, Zitronensäure, Weinsäure.

Erfindungsgemäß erhält man die neuen acylierten Diazepinone der allgemeinen Formel I nach folgenden Verfahren:

a.) durch Umsetzung von basisch substituierten kondensierten Diazepinonen der allgemeinen Formel II

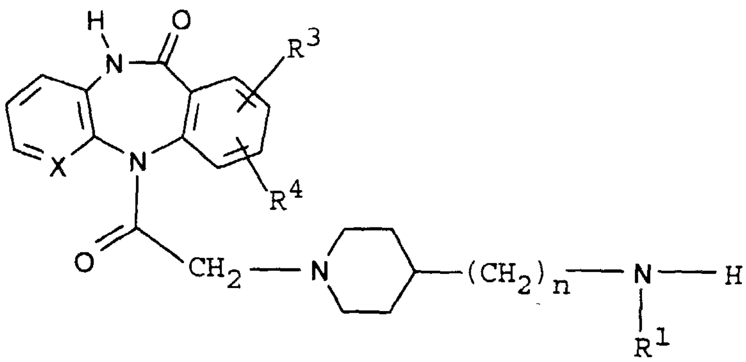

(II)

in der
X, $R^1$, $R^3$, $R^4$ und n wie eingangs erwähnt definiert sind, mit Carbonsäuren der allgemeinen Formel III

$$R^2 - \overset{\displaystyle O}{\overset{\|}{C}} - OH \qquad (III),$$

in der
$R^2$ wie oben erwähnt definiert ist, oder mit deren reaktionsfähigen Derivaten.

Als reaktionsfähige Derivate einer Carbonsäure der allgemeinen Formel III kommen beispielsweise deren Ester wie der Methyl-, Äthyl- oder Benzylester, deren Thioester wie der Methylthio- oder Äthylthioester, deren Halogenide wie das Säurechlorid, deren Anhydride oder Imidazolide in Betracht.

Die Umsetzung wird zweckmäßigerweise in einem Lösungsmittel wie Methylenchlorid, Chloroform, Tetrachlorkohlenstoff, Äther, Tetrahydrofuran, Dioxan, Benzol, Toluol, Acetonitril oder Dimethylformamid, gegebenenfalls in Gegenwart eines die Säure aktivierenden Mittels oder eines wasserentziehenden Mittels, z.B. in Gegenwart von Chlorameisensäureäthylester, Thionylchlorid, Phosphortrichlorid, Phosphorpentoxid, N,N'-Dicyclohexylcarbodiimid, N,N'-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, und gegebenenfalls in Gegenwart einer anorganischen Base wie Natriumcarbonat oder einer tertiären organischen Base wie Triäthylamin oder Pyridin, welche gleichzeitig als Lösungsmittel dienen können, bei Temperaturen zwischen -25 und 150°C, vorzugsweise jedoch bei Temperaturen zwischen -10°C und der Siedetemperatur des verwendeten Lösungsmittels, durchgeführt. Die Umsetzung kann auch ohne Lösungsmittel durchgeführt werden, desweiteren kann während der Umsetzung entstehendes Wasser durch azeotrope Destillation, z.B. durch Erhitzen mit Toluol am Wasserabscheider, oder durch Zugabe eines Trockenmittels wie Magnesiumsulfat oder Molekularsieb abgetrennt werden.

Beispielsweise werden die Umsetzungen mit einem Säurehalogenid in inerten Lösungsmitteln wie Ether, Toluol, Methylenchlorid und dergleichen, bei Temperaturen zwischen -50°C und bis zum Siedepunkt des Reaktionsgemisches, vorzugsweise zwischen 0° und 50°C, und vorzugsweise in Gegenwart eines halogenwasserstoffbindenden Mittels, z. B. eines tertiären Amins, Natriumcarbonat oder Calciumcarbonat, durchgeführt. Dabei können nicht nur die freien Amine der allgemeinen Formel II eingesetzt werden, sondern auch deren Salze, aus denen in situ die Amine durch die zugesetzten Hilfsbasen freigesetzt werden.

Beispielsweise werden die Umsetzungen in Gegenwart von Imidazoliden oder Carbodiimiden in einem hochsiedenden Lösungsmittel, wie Xylol, bei Rückflußtemperatur durchgeführt.

b) durch Acylierung von Diazepinonen der allgemeinen Formel IV

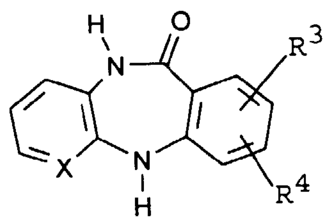

(IV),

worin

X, $R^3$ und $R^4$ die oben angegebenen Bedeutungen haben, mit Carbonsäurederivaten der allgemeinen Formel V

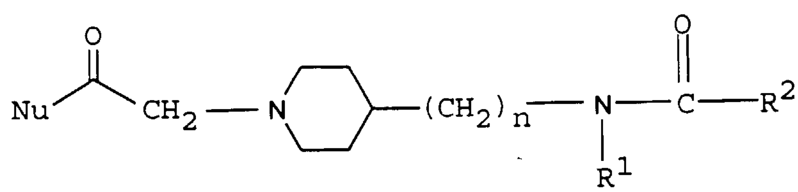

(V),

in der

n, $R^1$ und $R^2$ wie oben definiert sind und

Nu eine nucleofuge Gruppe bzw. Abgangsgruppe darstellt.

Die Umsetzung der Verbindungen der allgemeinen Formel IV mit den Säurederivaten der allgemeinen Formel V erfolgt in an sich bekannter Weise. Die Abgangsgruppe Nu ist eine Gruppe, die gemeinsam mit der Carbonylgruppe, an die sie gebunden ist, ein reaktives Carbonsäurederivat bildet. Als reaktive Carbonsäurederivate seien beispielsweise Säurehalogenide, -ester, -anhydride oder gemischte Anhydride, wie sie aus Salzen der entsprechenden Säuren (Nu=OH) und Säurechloriden, wie Phosphoroxidchlorid, Diphosphorsäuretetrachlorid oder Chlorameisensäureestern gebildet werden oder die bei der Umsetzung von Verbindungen der allgemeinen Formel V (Nu=OH) mit N-Alkyl-2-halogenpyridiniumsalzen entstehenden N-Alkyl-2-acyloxypyridiniumsalze genannt.

Bevorzugt wird die Reaktion mit den gemischten Anhydriden starker Mineralsäuren, insbesondere der Dichlorphosphorsäure, durchgeführt. Die Reaktion wird gegebenenfalls in Gegenwart eines säurebindenden Mittels (Protonenakzeptors) durchgeführt. Als geeignete Protonenakzeptoren seien beispielsweise Alkalimetallcarbonate oder -hydrogencarbonate, wie Natriumcarbonat oder Kaliumhydrogencarbonat; tertiäre organische Amine, wie Pyridin, Triethylamin, Ethyldiisopropylamin, 4-(Dimethylamino)pyridin, oder Natriumhydrid genannt. Die Reaktion wird bei Temperaturen zwischen -25°C und 130°C in einem inerten Lösungsmittel durchgeführt. Als inerte Lösungsmittel kommen beispielsweise chlorierte aliphatische Kohlenwasserstoffe, wie Methylenchlorid, 1,2-Dichlorethan; offenkettige oder cyclische Ether, wie Diethylether, Tetrahydrofuran oder 1,4-Dioxan; aromatische Kohlenwasserstoffe, wie Benzol, Toluol, Xylol, o-Dichlorbenzol; polare aprotische Lösungsmittel wie Acetonitril, Dimethylformamid oder Hexamethylphosphorsäuretriamid; oder Gemische davon in Frage. Die Reaktionszeiten betragen je nach Menge und Art des eingesetzten Acylierungsmittels der allgemeinen Formel V zwischen 15 Minuten und 80 Stunden. Es ist nicht nötig, die Verbindungen der allgemeinen Formel V in reiner Form herzustellen, sie können vielmehr im Reaktionsansatz in bekannter Weise in situ erzeugt werden.

Die so erhaltenen Basen der allgemeinen Formel I können anschließend in ihre Säureadditionssalze oder erhaltene Säureadditionssalze in die freien Basen oder andere pharmakologisch verträgliche Säureadditionssalze übergeführt werden.

Die erfindungsgemäßen kondensierten Diazepinone der allgemeinen Formel I enthalten bis zu zwei unabhängige chirale Kohlenstoffatome. Als weiteres chirales Element ist der acylierte Tricyclus selbst anzusehen, der in zwei spiegelbildlichen Formen vorliegen kann. Von der Natur des Tricyclus hängt es ab, ob die Energiebarriere für eine Inversion an diesem Zentrum so hoch ist, daß die einzelnen Isomeren bei Zimmertemperatur stabil sind und isolierbar werden. Es hat sich gezeigt, daß bei Verbindungen der allgemeinen Formel I, worin X ein Stickstoffatom ist und die dem Diazepinon-Ring benachbarten Positionen unsubstituiert sind, die erforderliche Aktivierungsenergie so stark vermindert ist, daß Diastereomere bei Zimmertemperatur nicht mehr nachgewiesen geschweige denn präparativ isoliert werden können.

Die erfindungsgemäßen kondensierten Diazepinone der allgemeinen Formel I enthalten also bis zu drei Chiralitätselemente, von denen eines bei Zimmertemperatur unter Umständen nicht konfigurationsstabil ist. Solche Verbindungen können deshalb in mehreren diastereomeren und/oder jeweils als enantiomere (+)- und (-)-Formen auftreten. Die Erfindung umfaßt die einzelnen Isomeren ebenso wie ihre Gemische. Die Trennung der jeweiligen Diastereomeren gelingt auf Grund der unterschiedlichen physiko-chemischen Eigenschaften, z.B. durch fraktioniertes Umkristallisieren aus geeigneten Lösungsmitteln, durch Hochdruckflüssigkeitschromatographie, Säulenchromatographie oder gaschromatographische Verfahren.

Die Spaltung evtl. Razemate der Verbindungen der allgemeinen Formel I kann nach bekannten Verfahren durchgeführt werden, beispielsweise unter Verwendung einer optisch aktiven Säure, wie (+)- oder (-)-Weinsäure, oder eines Derivats davon, wie (+)- oder (-)-Diacetylweinsäure, (+)- oder (-)-Monomethyltartrat oder (+)-Camphersulfonsäure.

Nach einem üblichen Verfahren zur Isomerentrennung wird das Racemat einer Verbindung der allgemeinen Formel I mit einer der vorstehend angegebenen optisch aktiven Säuren in äquimolarer Menge in einem Lösungsmittel umgesetzt und die erhaltenen kristallinen diastereomeren Salze werden unter Ausnutzung ihrer verschiedenen Löslichkeit getrennt. Diese Umsetzung kann in jeder Art von Lösungsmittel durchgeführt werden, solange dieses einen ausreichenden Unterschied in der Löslichkeit der Salze aufweist. Vorzugsweise werden Methanol, Ethanol oder deren Gemische, beispielsweise im Volumenverhältnis 50:50, verwendet. Sodann wird jedes der diastereomeren Salze in Wasser gelöst, mit einer Base, wie Natriumcarbonat oder Kaliumcarbonat, neutralisiert und dadurch die entsprechende freie Verbindung in der (+)- oder (-)-Form erhalten.

Jeweils nur ein Enantiomeres bzw. ein Gemisch zweier optisch aktiver unter die allgemeine Formel I fallender diastereomerer Verbindungen wird auch dadurch erhalten, daß man die oben beschriebenen Synthesen mit nur einem entsprechenden Enantiomeren durchführt.

Eine Ausgangsverbindung der allgemeinen Formel II läßt sich beispielsweise wie folgt herstellen:

Ein (Aminoalkyl)pyridin der allgemeinen Formel VI

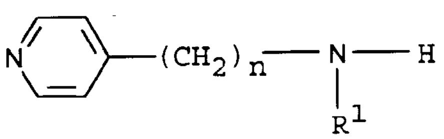 (VI),

in der
n und $R^1$ wie oben definiert sind (Verbindungen dieser Art sind literaturbekannt und können zum Teil käuflich erworben werden) wird in bekannter Weise tertiär-butoxycarbonyliert, wobei Verbindungen der allgemeinen Formel VII entstehen:

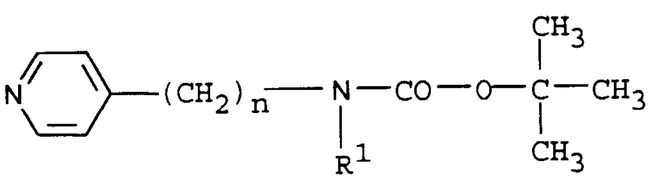 (VII),

Katalytische Hydrierung dieser Verbindungen nach bekannten Verfahren, z. B. in ethanolisch-salzsaurer Lösung und unter Verwendung von Platin(IV)-oxid als Katalysator (vgl. F.F. Blicke et al., J. Org. Chemistry 26, 3258 (1961)) oder in Eisessig in Gegenwart von Platin(IV)-oxid (vgl. W.F. Minor et al., J. Med. Pharm. Chem. 5, 96, 105ff (1962) und A. H. Sommers et al., J. Amer. chem. Soc., 75, 57, 58ff (1953)) ergibt Verbindungen der allgemeinen Formel VIII:

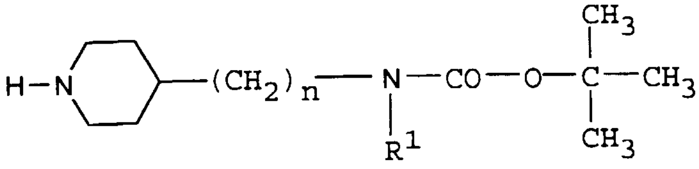 (VIII),

Eine solche Verbindung wird anschließend mit einer Halogenacylverbindung der allgemeinen Formel IX

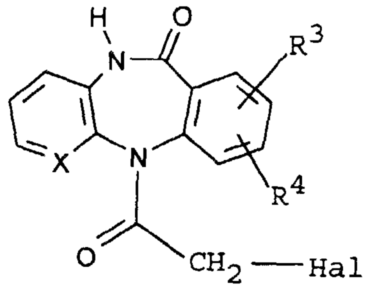

(IX),

in der

X, B und m wie oben definiert sind und Hal ein Chlor-, Brom- oder Jodatom bedeutet, umgesetzt.

Diese Aminierung erfolgt in einem inerten Lösungsmittel bei Temperaturen zwischen -10°C und der Siedetemperatur des Lösungsmittels, vorzugsweise entweder mit wenigstens 2 Molen sekundärem Amin der allgemeinen Formel VIII oder mit 1 bis 2 Molen des sekundären Amins der allgemeinen Formel VIII und einer Hilfsbase. Als Lösungsmittel kommen beispielsweise chlorierte Kohlenwasserstoffe, wie Methylenchlorid, Chloroform oder Dichlorethan; offenkettige oder cyclische Ether, wie Diethylether, Tetrahydrofuran oder Dioxan; aromatische Kohlenwasserstoffe, wie Benzol, Toluol, Xylol, Chlorbenzol oder Pyridin; Alkohole, wie Ethanol oder Isopropanol; Ketone, wie Aceton; Acetonitril, Dimethylformamid oder 1,3-Dimethyl-2-imidazolidinon in Frage. Als Hilfsbasen seien beispielsweise tertiäre organische Basen, wie Triethylamin, N-Methylpiperidin, Diethylanilin, Pyridin und 4-(Dimethylamino)pyridin oder anorganische Basen, wie Alkalimetall- oder Erdalkalimetallcarbonate oder -hydrogencarbonate, -hydroxide oder -oxide genannt. Gegebenenfalls kann die Reaktion durch Zusatz von Alkalimetalliodiden beschleunigt werden.

Man erhält hierbei eine Verbindung der allgemeinen Formel X

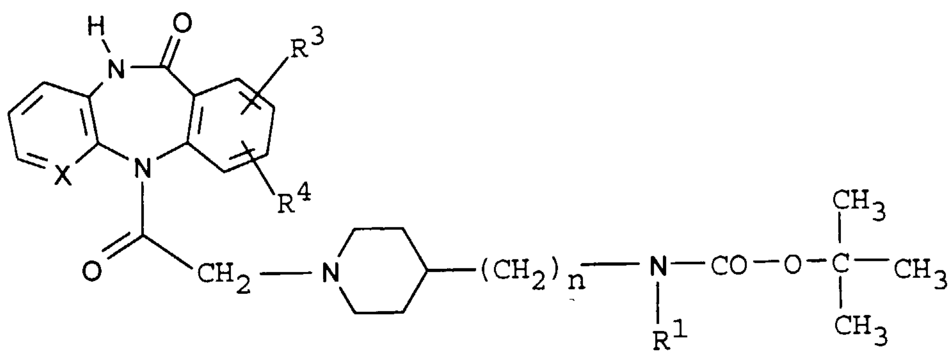

(X),

in der die Reste X, $R^1$, $R^3$, $R^4$ und n wie oben definiert sind, und aus der mit Hilfe z. B. von Bromwasserstoffsäure in Eisessig der tert. Butoxycarbonylrest in an sich bekannter Weise abgespalten wird, wodurch eine Verbindung der allgemeinen Formel II entsteht.

Die aktivierten Carbonsäuren der Formel III erhält man zweckmäßigerweise im Reaktionsgemisch nach literaturbekannten Methoden.

Die Diazepinone der allgemeinen Formel IV sind literaturbekannt (vgl. EP-A-0.039.519; EP-A-0.057.428; DE-C-1.179.943 und 1.204.680; F. Hunzicker et al., Arzneim.-Forsch. 13, 324 (1963)).

Die Carbonsäurederivate der allgemeinen Formel V, in der Nu eine Alkoxygruppe bedeutet, erhält man durch Umsetzung von substituierten Piperidinen der allgemeinen Formel VIII mit entsprechenden Halogenalkansäureestern gegebenenfalls unter Verwendung zusätzlicher Hilfsbasen, z. B. Triethylamin, oder Katalysatoren, z. B. Triton B. Durch Verseifung der erhaltenen Ester, z. B. mit Barytlauge, erhält man die unter die allgemeine Formel V fallenden Carbonsäuren, in denen Nu die Hydroxygruppe bedeutet. Die so erhaltenen Carbonsäuren dienen dann als Ausgangssubstanzen für die Herstellung von Derivaten mit anderen nucleofugen Gruppen, beispielsweise der Säurehalogenide der Formel V.

Ein weiterer Gegenstand der Erfindung sind Arzneimittel, die ein oder mehrere kondensierte Diazepinone der allgemeinen Formel I bzw. deren physiologisch verträgliche Salze enthalten.

Die Verbindungen der allgemeinen Formel I lassen sich hierzu in an sich bekannter Weise in die üblichen pharmazeutischen Zubereitungsformen, z.B. in Lösungen, Suppositorien, Tabletten, Dragees, Kapseln oder Teezubereitungen einarbeiten. Die Tagesdosis liegt im allgemeinen zwischen 0,02 und 5 mg/kg, vorzugsweise 0,02 und 2,5 mg/kg, insbesondere 0,05 und 1,0 mg/kg Körpergewicht, die gegebenenfalls in Form mehrerer, vorzugsweise 1 bis 3 Einzelgaben, zur Erzielung der gewünschten Ergebnisse verabreicht wird.

Wie bereits eingangs erwähnt, weisen die basisch substituierten kondensierten Diazepinone der allgemeinen Formel I und ihre Säureadditionssalze wertvolle Eigenschaften auf; so besitzen sie günstige Selektivitäten für cardiale $M_2$-Rezeptoren und können deshalb als vagale Schrittmacher zur Behandlung von Bradycardien und Bradyarrhythmien in der Human- und auch der Veterinärmedizin eingesetzt werden.

Aus Untersuchungen von van Charldorp und van Zwieten (siehe K.J. van Charldorp, Dissertation "Characterisation of Muscarinic Receptors in the Vascular System", Amsterdam 1988; K.J. van Charldorp, D. Davidesko und P.A. van Zwieten, Eur. J. Pharmacol. 150, 197-199 (1988); K.J. van Charldorp und P.A. van Zwieten, Naunyn Schmiedeberg's Arch. Pharmacol. 339, 403-408 (1989)) ist bekannt, daß die muscarinischen Rezeptoren in den Basilararterien, die für eine Kontraktion der Gefäße verantwortlich sind, vom $M_2$-Typ sind. Deshalb ist zu erwarten, daß $M_2$ Antagonisten den Tonus der cerebralen Blutgefäße erniedrigen und damit die Durchblutung steigern.

Hemmung der in Hirngefäßen nachweisbaren muscarinischen Rezeptoren führt also zu einer Vermeidung der Konstriktionen und zu einer Verbesserung bzw. Normalisierung der arteriosklerotisch bedingten Störungen der Cerebraldurchblutungen. Die erfindungsgemäßen neuen Verbindungen eignen sich besonders gut zur Verbesserung bzw. Normalisierung arteriosklerotisch bedingter Störungen der Cerebraldurchblutung.

Eine Reihe von Verbindungen der allgemeinen Formel I zeigt infolge hoher Lipophilie eine gute ZNS-Gängigkeit und eignet sich daher zusätzlich auch zur Therapie von Erkrankungen des Zentralnervensystems, insbesondere des Morbus Alzheimer. Bei seniler Demenz vom Alzheimer-Typ führt Degeneration cholinerger Neuronen, insbesondere in hippocampalen und corticalen Projektionen, zu verminderter Freisetzung des Neurotransmitters Acetylcholin. Die Blockade der präsynaptischen Autorezeptoren unterbricht nun den negativen Rückkopplungsmechanismus, den der Neurotransmitter an den noch intakten Neuronen ausübt, und bewirkt daher einen gesteigerten Acetylcholin-Release und - damit verknüpft - eine Stimulierung der postsynaptischen Rezeptoren (D.C. Mash, D L. Flynn und L.T. Potter, Science 228, 115-117 (1985); E.K. Perry u. a., Can. J. Neurol. Sci. 13, 521-527 (1986); M. Sarter u. a., TINS 11, 13-17 (1988)). Die Verbindungen eignen sich deshalb in der Geriatrie und fördern in signifikanter Weise die Lern- und Merkfähigkeit.

Zum Nachweis der günstigen Effekte auf die cerebrale Durchblutung wurden die folgenden Versuche durchgeführt:

A Bindungsstudien an muskarinischen Rezeptoren

Männliche Wistar-Ratten (Chbb: THOM Stamm, 180-220 g Körpergewicht) wurden durch Genickschlag getötet. Großhirnrinde, Herz und Speicheldrüsen wurden entfernt, gewaschen und jeweils im 20-fachen Volumen von HEPES-Puffer (20 mM 4-(2-Hydroxyethyl)-1-piperazinethansulfonsäure, 100 mM Natriumchlorid, 10 mM Magnesiumchlorid, pH 7,5) mittels eines Ultra-Turrax-Gerätes bei einer maximalen Umdrehung von 60 g homogenisiert. Die Homogenate wurden auf 1:500 verdünnt, bezogen auf die ursprüngliche Gewebsmenge. Für den Bindungsversuch wurde 1 nM [$^3$H] Pirenzepin (3,22 TBq/mmol), das an $M_1$-Rezeptoren der Cortex bindet und 0,3 nM [$^3$H]NMS ($^3$H-N-Methylscopolamin) (2,64 TBq/mmol) zur Bindung an Herz und Speicheldrüse bei Zimmertemperatur, für [$^3$H]Pirenzepin 90 Minuten, für [$^3$H]NMS 40 Minuten lang jeweils mit 0,35, 0,30 und 0,20 mg Protein pro Probe (0,5 ml) für Herz, Speicheldrüse und Cortex inkubiert. Die Proteinkonzentration wurde nach der Methode von Lowry et al. (J. Biol. Chem. 93, 265) bestimmt. Die Inkubation wurde durch rasche Filtration durch eine Glasfaserfiltermatte und Benutzung eines Skatron Cell Harvesters beendet. Nach zweimaliger Waschung (10-Sekunden-Waschung mit ca. 3 ml Flüssigkeit) wurden die Filter an der Luft getrocknet, in Minivialen abgefüllt, über Nacht mit 4 ml Scintillationsflüssigkeit geschüttelt und mit einer Wirksamkeit von 45-50 % mittels eines Packard 460C-Gerätes die Zählung der Scintillation vorgenommen. Alle Versuche wurden 3-fach durchgeführt. Die nicht-spezifische Bindung wurde definiert als die Radioaktivität in Gegenwart von 1 µM (-)-3-Chinuclidinyl-benzilat. Die Bindungsdaten wurden durch eine komputerunterstützte nicht-lineare "least-square curve fitting"-Methode (Heinzel, G., 1982, in: Pharmacokinetics during Drug Development: Data Analysis and Evaluation Techniques, eds. G. Bozler and J.M. van Rossum (Gustav-Springer-Verlag), Seite 207) analysiert. Die Dissoziationskonstanten Ki wurden aus den $IC_{50}$-Werten nach den Angaben von Cheny und Prusoff (Biochem. Pharmacol. 22, 3099 (1973)) berechnet. Die Ergebnisse sind in der Tabelle 1 zusammengefaßt.

B Untersuchung auf zentrale Wirkung

Prinzip:

Arecolin hat sowohl eine zentrale als auch eine peripher wirksame Komponente. Die peripheren (blutdrucksenkenden) Effekte werden durch N-Methylscopolamin blockiert, sodaß nur noch der zentrale (blutdrucksteigernde) Effekt von Arecolin auftritt. Gehirngängige Antimuskarinika blockieren diesen zentrale Effekt.

Methode:

Männliche 300 g schwere Ratten werden mit Urethan (1,2 g/kg) i.p. narkotisiert. Die Trachea wird inkubiert, die

Tiere werden mit einem Luft/Sauerstoff-Gemisch beatmet (80 Hübe/ min). Der Blutdruck wird nach dem Kanülieren der A. carotis über einen Druckaufnehmer (Bell und Howell Type 4-327-I) registriert. Die Testsubstanzen werden über die V.jugularis appliziert (0,5 mol/kg). Vor Versuchsbeginn erhalten die Tiere 0,5 mg/kg N-Methyl-scopolamin. Die Arecolinapplikationen (jeweils 0,3 mg/kg) erfolgen in 15-Minuten-Abständen. Nach 2 Vergleichswerten wird die Testsubstanz in steigender Dosierung jeweils 5 Minuten vor der erneuten Arecolinapplikation injiziert.

Die erzielten Ergebnisse wurden mit den gemittelten Ausgangswerten verglichen und in % Hemmung ermittelt. Die Tabelle 2 enthält die beim Arecolin-Test gefundenen Werte.

C Einfluß auf das Gedächtnis beim Morris Water Maze

Die Untersuchungen wurden unter Verwendung des von R.G.M. Morris in Learn Motive 12, 239-249 (1981) beschriebenen Wasser-Labyrinths nach der dort geschilderten Versuchsdurchführung vorgenommen.

Am 1. Tag erhielten 24 Monate alte und damit lernbehinderte Ratten (n = 7/Gruppe) 0,2 mg/kg der Verbindung D subcutan, eine andere Gruppe dieser Tiere physiologische Kochsalzlösung (s.c.) verabreicht. Die Gruppen wurden 4 Tage lang im Morris Water Maze beobachtet.

Wie sich aus dem Verlauf der dabei gewonnenen Kurven der Figur 1 ergibt, bewirkt die Verabreichung der Verbindung D eine verbesserte Gedächtnisleistung im Vergleich zu der der nur mit physiologischer Kochsalzlösung behandelten, aus Altersgründen lernbehinderten Tiere. So wurde z. B. am 2. Tag die Latenzzeit um annähernd 50% gesenkt. Am 4. Tag erlangten die mit Substanz D behandelten Tiere dasselbe Niveau wie die Kontrollgruppen junger Ratten (n = 7/Gruppe), die nur mit der physiologischen Kochsalzlösung behandelt wurden. Diese Wirkung der erfindungsgemäßen $M_2$-Antagonisten ist überraschend, da für den $M_1$-selektiven Antagonisten Pirenzepine eine Verschlechterung des Lernvermögens und der Gedächtnisleistung beobachtet wurde (A.J. Hunter und F.F. Roberts, Pharmacol. Biochem. and Behavior Vol. 30, 519-523 (1988), Titel: "The effects of pirenzepine on spatial learning in the Morris Water Maze"). Von ähnlichen negativen Beobachtungen bei nicht selektiven Substanzen wie Scopolamin oder Atropin berichten auch A.J. Hunter, F.F. Roberts und C.A. Tutty in Br. J. Pharmacol. 87, 41P, 1986; R.J. Sutherland, I. Q. Wishaw und J.C. Regehr, J. Comp. Physiol. Psychol. 96, 563-573 (1982) und I.Q. Wishaw, Behav. Neurosci. 99, 979-1005 (1985).

Nach den vorstehenden Angaben wurden beispielsweise die folgenden Verbindungen untersucht:

A = 5,11-Dihydro-11-[[4-[3-[(2,2-dimethyl-1-oxobutyl]ethyl-amino]-propyl]-1-piperidinyl]acetyl]-6H-pyrido[2,3-b]-[1,4]benzodiazepin-6-on

B = 5,11-Dihydro-11-[[4-[3-[(2,2-dimethyl-1-oxopentyl)ethylamino]-propyl]-1-piperidinyl]acetyl]-6H-pyrido[2,3-b]-[1,4]benzodiazepin-6-on

C = 5,11-Dihydro-11-[[4-[4-[(2,2-dimethyl-1-oxopentyl)ethylamino]butyl]-1-piperidinyl]acetyl]-6H-pyrido[2,3-b] [1,4]-benzodiazepin-6-on

D = 5,11-Dihydro-8-chlor-11-[[4-[3-[(2,2-dimethyl-1-oxopentyl)-ethylamino]propyl]-1-piperidinyl]acetyl]-6H-pyrido-[2,3-b][1,4]benzodiazepin-6-on

E = 5,11-Dihydro-8-methyl-11-[[4-[3-[(2,2-dimethyl-1-oxopropyl)-ethylamino]propyl]-1-piperidinyl]acetyl]-6H-pyrido-[2,3-b] [1,4]benzodiazepin-6-on

F = 5,11-Dihydro-11-[[4-[3-[(2,2-dimethyl-1-oxopropyl)ethylamino]propyl]-1-piperidinyl]acetyl]-6H-pyrido[2,3-b]-[1,4]-benzodiazepin-6-on

G = 5,11-Dihydro-8-methyl-11-[[4-[3-[(2,2-dimethyl-1-oxobutyl)ethylamino]propyl]-1-piperidinyl]acetyl]-6H-pyrido-[2,3-b][1,4]benzodiazepin-6-on

und mit den folgenden bekannten Substanzen verglichen:

H=11-[[2-[(Diethylamino)methyl]-1-piperidinyl]acetyl]-5,11-dihydro-6H-pyrido[2,3-b][1,4]benzodiazepin-6-on (siehe US-Patent Nr. 4,550,107)

I = 11-[[4-[4-(Diethylamino)butyl]-1-piperidinyl]acetyl]-5,11-dihydro-6H-pyrido[2,3-b][1,4]benzodiazepin-6-on (siehe EP-A2-0 312 895)

aus EP-A1-0 402 734:

J = 5,11-Dihydro-11-[1-oxo-6-(1-piperidinyl)-4-hexin-1-yl]-6H-pyrido[2,3-b][1,4] benzodiazepin-6-on

K=(±)-9-Chlor-11-[[2-[(diethylamino)methyl]-1-piperidinyl]-acetyl]-5,11-dihydro-6H-pyrido[2,3-b][1,4]benzodiazepin-6-on

L = 5,11-Dihydro-11-[[[2-[2-[(dipropylamino)methyl]-1-piperidinyl]ethyl]amino]carbonyl]-6H-pyrido[2,3-b][1,4]benzodiazepin-6-on-methansulfonat

M = 5,11-Dihydro-11-[[3-[3-(1-piperidinyl)-1-propyl]-1-piperidinyl]carbonyl]-6H-pyrido[2,3-b][1,4]benzodiazepin-6-on

N = 4,9-Dihydro-3-methyl-4-[[4-[3-(1-piperidinyl)-1-propyl]-1-piperidinyl]acetyl]-10H-thieno[3,4-b][1,5]benzodiazepin-10-on

O = 5,11-Dihydro-11-[1-oxo-6-(1-piperidinyl)-1-hexyl]-6H-pyrido[2,3-b][1,4]benzodiazepin-10-on

P = 4,9-Dihydro-3-methyl-4-[6-(hexahydro-1H-1-azepinyl)-1-oxo-4-hexin-1-yl]-10H-thieno[3,4-b][1,5]benzodiazepin-10-on

Q = 11-[4-[3-[(Diethylamino)methyl]-4-morpholinyl]-1-oxo-1-butyl]-5,11-dihydro-6H-pyrido[2,3-b] [1,4]benzodiazepin-6-on

R = 5,11-Dihydro-11-[[[2-(1-methyl-2-pyrrolidinyl)ethyl]-methylamino]acetyl]-6H-pyrido[2,3-b][1,4]benzodiazepin-6-on

S = 5,11-Dihydro-11-[[[2-(1-methyl-hexahydro-1H-2-azepinyl)-ethyl]methylamino]acetyl]-6H-pyrido[2,3-b][1,4] benzodiazepin-6-on

Die Ergebnisse sind in den folgenden Tabellen 1 und 2 wiedergegeben:

Tabelle 1

| Rezeptor-Bindungstests, in vitro: | | | | |
|---|---|---|---|---|
| Ergebnisse: | | | | |
| Substanz | Rezeptor-Bindungs-Tests Ki [nM] | | | Selektivitätsverhältnis ($M_1/M_2$) |
| | $M_1$ (Cortex) | $M_2$ (Herz) | $M_3$ (Submandibularis) | |
| A | 500 | 25 | 750 | 20 |
| B | 150 | 15 | 300 | 10 |
| C | 500 | 38 | 875 | 13 |
| D | 866 | 25 | 916 | 35 |
| E | 1000 | 100 | 1500 | 10 |
| F | 900 | 65 | 1500 | 14 |
| G | 600 | 25 | 750 | 24 |
| H | 500 | 79 | 1995 | 6 |
| I | 20 | 5 | 151 | 4 |
| J | 794 | 120 | 1820 | 6,6 |
| K | 126 | 25 | 759 | 5 |
| L | 30 | 7,9 | 100 | 3,8 |
| M | 13 | 2 | 13 | 6,5 |
| N | 7,9 | 2 | 6 | 4 |
| O | 10 | 5 | 40 | 2 |
| P | 35 | 7,6 | 30 | 4,6 |

Tabelle 1 (fortgesetzt)

| Rezeptor-Bindungstests, in vitro: | | | | |
|---|---|---|---|---|
| Ergebnisse: | | | | |
| Substanz | Rezeptor-Bindungs-Tests Ki [nM] | | | Selektivitätsverhältnis ($M_1/M_2$) |
| | $M_1$ (Cortex) | $M_2$ (Herz) | $M_3$ (Submandibularis) | |
| Q | 50 | 20 | 50 | 2,5 |
| R | 7,1 | 3 | 30 | 2,4 |
| S | 16 | 3 | 25 | 5,3 |

Tabelle 2

| ZNS-Penetration, in vivo (Ratte) | |
|---|---|
| Substanz | Abnahme der durch Arecolin induzierten Blutdrucksteigerung $ED_{50}$ [mg/kg] |
| A | 4,5 |
| B | 1,3 |
| C | 2,0 |
| D | 5,0 |
| E | 2,8 |
| F | 6,5 |
| G | 5,1 |
| H | >10,0 |
| K | >10,0 |
| L | >10,0 |
| O | >10,0 |

Wie aus der Tabelle 1 hervorgeht, zeigen die genannten Verbindungen A bis G ausgezeichnete $M_1/M_2$-Selektivitäten im Bereich von 10-35. Dieses Verhältnis besagt, daß die genannten Verbindungen hoch $M_2$-selektiv und deshalb in der Lage sind, präsynaptische $M_2$-Rezeptoren im Gehirn zu blockieren, und zwar in einem Dosisbereich, in dem $M_1$-Rezeptoren noch nicht beeinflußt werden. Im Gegensatz hierzu besitzen die Vergleichssubstanzen H bis S nur mäßig ausgeprägte $M_1/M_2$-Selektivitäten mit einem Selektivitätsverhältnis < 6.6. Ein Vergleich dieser Selektivitätswerte zeigt, daß die neuen Diazepinone der allgemeinen Formel den Vergleichssubstanzen weitaus überlegen sind.

Eine wesentliche Voraussetzung für die Anwendbarkeit der Verbindungen zur Behandlung von ZNS-Erkrankungen ist die Gehirngängigkeit der Substanzen. Das vorbeschriebene Prüfmodell zum Nachweis der ZNS-Penetration zeigt eindeutig (siehe Tabelle 2), daß die Substanzen A bis G die durch Arecolin vermittelten zentralen Effekte zu hemmen vermögen. Dieser Befund kann nur auf der Basis einer guten ZNS-Gängigkeit erklärt werden. Im Gegensatz hierzu, zeigen die untersuchten Vergleichssubstanzen H, K, L und O mit $ED_{50}$-Werten > 10 mg/kg (i.v.) keine Effekte auf die durch Arecolin vermittelte Blutdrucksteigerung. Damit ist eindeutig bewiesen, daß diese Verbindungen eine wesentlich schlechtere Penetration in das Zentralnervensystem besitzen.

Die nachfolgenden Beispiele sollen die Erfindung näher erläutern:

## Beispiel 1

5,11-Dihydro-11-[[4-[3-[(2,2-dimethyl-1-oxobutyl)ethylamino]-propyl]-1-piperidinyl]acetyl]-6H-pyrido[2,3-b][1,4]benzodiazepin-6-on

1,48 g (0,01 Mol) 2,2-Dimethylbuttersäurechlorid gelöst in 20 ml Tetrahydrofuran werden unter Rühren bei Raumtemperatur zu einer Lösung von 4,2 g (0,01 Mol) 5,11-Dihydro-11-[[4-[3-(ethylamino)propyl]-1-piperidinyl]acetyl]-6H-pyrido-[2,3-b][1,4]benzodiazepin-6-on und 2 ml Triethylamin in 150 ml Tetrahydrofuran zugetropft. Zur Vervollständigung der Reaktion wird die Lösung noch eine Stunde bei 50°C weiter gerührt. Nach dem Abkühlen wird vom ausgefallenen Triethylaminhydrochlorid abfiltriert und das Filtrat am Rotationsverdampfer im Wasserstrahlvakuum zur Trokkene eingeengt. Zur Reinigung löst man den Rückstand in Essigsäureethylester. Man schüttelt die Essigsäureethylester-Lösung zweimal mit 10%-iger Salzsäure aus, trennt die organische Phase ab und stellt die wässrige Phase durch

Zugabe von konzentriertem Ammoniak alkalisch. Nachfolgend wird die wässrige Phase zweimal mit Essigsäureethylester extrahiert. Man trocknet die organische Phase über Natriumsulfat und engt im Vakuum zur Trockene ein. Der erhaltene Rückstand wird durch Digerieren in Essigsäureethylester zur Kristallisation gebracht. Man erhält farblose Kristalle vom Fp. 136-138°C.
Ausbeute: 2,4 g (46,2 % der Theorie).

Beispiel 2

5,11-Dihydro-11-[[4-[3-[(2,2-dimethyl-1-oxo-propyl)ethylamino]propyl]-1-piperidinyl]acetyl]-6H-pyrido[2,3-b][1,4]-benzodiazepin-6-on

Hergestellt analog Beispiel 1 aus 5,11-Dihydro-11-[[4-[3-(ethylamino)propyl]-1-piperidinyl]acetyl]-6H-pyrido-[2,3-b][1,4]benzodiazepin-6-on und Pivalinsäurechlorid in einer Ausbeute von 61 % der Theorie.
Farblose Kristalle vom Fp. 154-155°C (Essigsäureethylester).

Beispiel 3

5,11-Dihydro-11-[[4-[3-[(2,2-dimethyl-1-oxopentyl)ethyl-amino]propyl]-1-piperidinyl]acetyl]-6H-pyrido[2,3-b][1,4]-benzodiazepin-6-on

Hergestellt analog Beispiel 1 aus 5,11-Dihydro-11-[[4-[3-(ethylamino)propyl]-1-piperidinyl]acetyl]-6H-pyrido-[2,3-b][1,4]benzodiazepin-6-on und 2,2-Dimethylvaleriansäurechlorid in einer Ausbeute von 62 % der Theorie.
Farblose Kristalle vom Fp. 138-140°C (Diethylether).

Beispiel 4

5,11-Dihydro-8-chlor-11-[[4-[3-[(2,2-dimethyl-1-oxopropyl)ethylamino]propyl]-1-piperidinyl]acetyl]-6H-pyrido-[2,3-b][1,4]benzodiazepin-6-on

Hergestellt analog Beispiel 1 aus 5,11-Dihydro-8-chlor-11-[[4-[3-(ethylamino)propyl]-1-piperidinyl]acetyl]-6H-pyrido[2,3-b][1,4]benzodiazepin-6-on und Pivalinsäurechlorid in einer Ausbeute von 77 % der Theorie.
Farblose Kristalle vom Fp. 168-170°C (Essigsäureethylester).

Beispiel 5

5,11-Dihydro-8-chlor-11-[[4-[3-[(2,2-dimethyl-1-oxobutyl)-ethylamino]propyl]-1-piperidinyl]acetyl]-6H-pyrido-[2,3-b][1,4]benzodiazepin-6-on

Hergestellt analog Beispiel 1 aus 5,11-Dihydro-8-chlor-11-[[4-[3-(ethylamino)propyl]-1-piperidinyl]acetyl]-6H-pyrido-[2,3-b] [1,4]benzodiazepin-6-on und 2,2-Dimethylbuttersäurechlorid in einer Ausbeute von 36 % der Theorie.
Farblose Kristalle vom Fp. 173-174°C (Diisopropylether).

Beispiel 6

5,11-Dihydro-8-chlor-11-[[4-[3-[(2,2-dimethyl-1-oxopentyl)-ethylamino]propyl]-1-piperidinyl]acetyl]-6H-pyrido-[2,3-b] [1,4]benzodiazepin-6-on

Hergestellt analog Beispiel 1 aus 5,11-Dihydro-8-chlor-11-[[4-[3-(ethylamino)propyl]-1-piperidinyl]acetyl]-6H-pyrido-[2,3-b] [1,4]benzodiazepin-6-on und 2,2-Dimethylvaleriansäurechlorid in einer Ausbeute von 70 %.
Farblose Kristalle vom Fp. 172-173°C (Essigsäureethylester).

Beispiel 7

5,11-Dihydro-8-chlor-11-[[4-[3-[(cyclopropylcarbonyl)ethylamino]propyl]-1-piperidinyl]acetyl]-6H-pyrido[2,3-b][1,4]-benzodiazepin-6-on

Hergestellt analog Beispiel 1 aus 5,11-Dihydro-8-chlor-11-[[4-[3-(ethylamino)propyl]-1-piperidinyl]acetyl]-6H-pyrido-[2,3-b][1,4] benzodiazepin-6-on und Cyclopropancarbonsäurechlorid in einer Ausbeute von 76 % der Theorie.

Farblose Kristalle vom Fp. 136-138°C (Diethylether/Essigsäureethylester).

Beispiel 8

5,11-Dihydro-8-methyl-11-[[4-[3-[(2,2-dimethyl-1-oxopropyl)-ethylamino]propyl]-1-piperidinyl]acetyl]-6H-pyrido-[2,3-b] [1,4]benzodiazepin-6-on

Hergestellt analog Beispiel 1 aus 5,11-Dihydro-8-methyl-11-[[4-[3-(ethylamino)propyl]-1-piperidinyl]acetyl]-6H-pyrido-[2,3-b] [1,4]benzodiazepin-6-on und Pivalinsäurechlorid in einer Ausbeute von 23 % der Theorie.
Farblose Kristalle vom Fp. 177-179°C (Acetonitril).

Beispiel 9

5,11-Dihydro-9-chlor-11-[[4-[3-[(2,2-dimethyl-1-oxopentyl)-ethylamino]propyl]-1-piperidinyl]acetyl]-6H-pyrido-[2,3-b] [1,4]benzodiazepin-6-on

Hergestellt analog Beispiel 1 aus 5,11-Dihydro-9-chlor-11-[[4-[3-(ethylamino)propyl]-1-piperidinyl]acetyl]-6H-pyrido-[2,3-b] [1,4]benzodiazepin-6-on und 2,2-Dimethylvaleriansäurechlorid. Die Reinigung erfolgte durch Chromatographie an Kieselgel (Firma Merck, 30-60 µm) mit einer Mischung von Essigsäureethylester/Ammoniak (10:0,1) als Elutionsmittel.
Ausbeute: 48 % der Theorie.
Farblose Kristalle vom Fp. 150-152°C (Diethylether).

Beispiel 10

5,11-Dihydro-9-chlor-11-[[4-[3-[(2,2-dimethyl-1-oxobutyl)-ethylamino]propyl]-1-piperidinyl]acetyl]-6H-pyrido-[2,3-b] [1,4]benzodiazepin-6-on

Hergestellt analog Beispiel 1 aus 5,11-Dihydro-9-chlor-11-[[4-[3-(ethylamino)propyl]-1-piperidinyl]acetyl]-6H-pyrido-[2,3-b] [1,4]benzodiazepin-6-on und 2,2-Dimethylbuttersäurechlorid. Die Reinigung der Base erfolgte durch Chromatographie an Kieselgel (Firma Merck, 30-60 µm) mit einer Mischung von Essigsäureethylester/Ammoniak (10:0,1).
Ausbeute: 54 % der Theorie.
Farblose Kristalle vom Fp. 123-125°C (Diethylether).

Beispiel 11

5,11-Dihydro-8-methyl-11-[[4-[3-[(2,2-dimethyl-1-oxobutyl)ethylamino]propyl]-1-piperidinyl]acetyl]-6H-pyrido-[2,3-b] [1,4]benzodiazepin-6-on

Hergestellt analog Beispiel 1 aus 5,11-Dihydro-8-methyl-11-[[4-[3-(ethylamino)propyl]-1-piperidinyl]acetyl]-6H-pyrido-[2,3-b][1,4]benzodiazepin-6-on und 2,2-Dimethylbuttersäurechlorid. Die Reinigung erfolgte durch Chromatographie an Kieselgel (Firma Baker, 30-60 µm) mit einer Mischung von Essigsäureethylester/Ammoniak (10:0,1) als Elutionsmittel.
Ausbeute: 41 % der Theorie.
Farblose Kristalle vom Fp. 97-99°C (Essigsäureethylester).

Beispiel 12

5,11-Dihydro-8-methyl-11-[[4-[3-[(2,2-dimethyl-1-oxopentyl)ethylamino]propyl]-1-piperidinyl]acetyl]-6H-pyrido-[2,3-b] [1,4]benzodiazepin-6-on

Hergestellt analog Beispiel 1 aus 5,11-Dihydro-8-methyl-11-[[4-[3-(ethylamino)propyl]-1-piperidinyl]acetyl]-6H-pyrido[2,3-b][1,4]benzodiazepin-6-on und 2,2-Dimethylvaleriansäurechlorid. Reinigung durch Chromatographie an Kieselgel (Firma Baker, 30-60 µm) mit einer Mischung von Essigsäureethylester/Ammoniak (10:0,1) als Elutionsmittel.
Ausbeute: 28 % der Theorie.
Farblose Kristalle vom Fp. 83-85°C (Essigsäureethylester).

Beispiel 13

5,11-Dihydro-8-chlor-11-[[4-[3-[(cyclohexylcarbonyl)ethylamino]propyl]-1-piperidinyl]acetyl]-6H-pyrido [2,3-b][1,4]-benzodiazepin-6-on

Hergestellt analog Beispiel 1 aus 5,11-Dihydro-8-chlor-11-[[4-[3-(ethylamino)propyl]-1-piperidinyl]acetyl]-6H-pyrido-[2,3-b][1,4]benzodiazepin-6-on und Cyclohexancarbonsäurechlorid. Die Reinigung erfolgte durch Chromatographie an Kieselgel (Firma Baker, 30-60 μm) mit einer Mischung von Essigsäureethylester/Methanol/Cyclohexan/Ammoniak = 8:1:1:0,1 als Elutionsmittel.
Ausbeute: 70 % der Theorie.
Farblose Kristalle vom Fp. 162-163°C (Diethylether).

Beispiel 14

5,11-Dihydro-8-chlor-11-[[4-[3-[(1-methylcyclohexylcarbonyl)ethylamino]propyl-1-piperidinyl]acetyl]-6H-pyrido-[2,3-b][1,4]benzodiazepin-6-on

Eine Lösung von 313 mg (2,2 mMol) 1-Methylcyclohexancarbonsäure und 340 mg (3,0 mMol) N.N'-Carbonyldiimidazol in 20 ml Tetrahydrofuran wird unter Rühren 1 Stunde auf 45°C erwärmt. Danach gibt man 920 mg (2 mMol) 5,11-Dihydro-8-chlor-[[4-[3-(ethylamino)propyl]-1-piperidinyl]acetyl]-6H-pyrido[2,3-b]-[1,4]benzodiazepin-6-on hinzu und rührt die Reaktionslösung bei 45°C noch zwei Stunden weiter. Nach beendeter Reaktion gießt man die Reaktionsmischung in eine gesättigte Natriumchlorid-Lösung ein, trennt die organische Phase ab und engt im Vakuum zur Trockene ein. Der erhaltene Rückstand wird zwischen Wasser und Essigsäureethylester verteilt und die Essigsäureethylester-Phase anschließend im Vakuum eingedampft. Das erhaltene Rohprodukt wird an Kieselgel (Firma Baker, 30-60 μm) unter Verwendung von einem Gemisch bestehend aus Essigsäureethylester/Methanol/Ammoniak (9:1:0,1) chromatographisch gereinigt. Man erhält die gewünschte Verbindung als amorphes Produkt in einer Ausbeute von 130 mg (11 % der Theorie).
$R_F$-Wert im Dünnschicht-Chromatogramm: 0,4 (DC-Platten: Kieselgel, Firma Merck; Fließmittel: Methylenchlorid/Cyclohexan/Methanol/Ammoniak = 680:150:150:20).

Beispiel 15

5,11-Dihydro-8-chlor-11-[[4-[3-[(tricyclo[3,3,1,1$^{3,7}$]dec-1-ylcarbonyl)ethylamino]propyl]-1-piperidinyl]acetyl]-6H-pyrido [2,3-b] [1,4]benzodiazepin-6-on

Hergestellt analog Beispiel 1 aus 5,11-Dihydro-8-chlor-11-[[4-[3-(ethylamino)propyl]-1-piperidinyl]acetyl]-6H-pyrido-[2,3-b] [1,4]benzodiazepin-6-on und Tricyclo[3,3,1,1$^{3,7}$]decan-1-carbonsäurechlorid in einer Ausbeute von 20 % der Theorie. Reinigung durch Chromatographie an Kieselgel (Firma Baker) mit einem Gemisch aus Essigsäureethylester/Cyclohexan/Methanol/Ammoniak = 80:10:10:1 als Elutionsmittel.
$R_F$-Wert im Dünnschichtchromatogramm: 0,65 (DC-Platten: Kieselgel, Firma Merck; Fließmittel: Methylenchlorid/Cyclohexan/Methanol/Ammoniak = 680:150:150:20).

Beispiel 16

5,11-Dihydro-11-[[4-[4-[(2,2-dimethyl-1-oxopropyl)ethylamino]butyl]-1-piperidinyl]acetyl]-6H-pyrido[2,3-b][1,4]-benzodiazepin-6-on

Hergestellt analog Beispiel 1 aus 5,11-Dihydro-11-[[4-[4(ethylamino)butyl]-1-piperidinyl]acetyl]-6H-pyrido-[2,3-b] [1,4]benzodiazepin-6-on und Pivalinsäurechlorid in einer Ausbeute von 62 % der Theorie.
Farblose Kristalle vom Fp. 206-207°C (Essigsäureethylester).

Beispiel 17

5,11-Dihydro-11-[[4-[4-[(cyclopropylcarbonyl)ethylamino]butyl]-1-piperidinyl]acetyl]-6H-pyrido[2,3-b][1,4]benzodiazepin-6-on

Hergestellt analog Beispiel 1 aus 5,11-Dihydro-11-[[4-[4-(ethylamino)butyl]-1-piperidinyl]acetyl]-6H-pyrido-[2,3-b] [1,4]benzodiazepin-6-on und Cyclopropancarbonsäurechlorid in einer Ausbeute von 67 % der Theorie.

Farblose Kristalle vom Fp. 202-204°C (Essigsäureethylester).

Beispiel 18

5,11-Dihydro-11-[[4-[4-[(2,2-dimethyl-1-oxobutyl)ethylamino]butyl] -1-piperidinyl]acetyl]-6H-pyrido[2,3-b][1,4]benzodiazepin-6-on

Hergestellt analog Beispiel 1 aus 5,11-Dihydro-11-[[4-[4(ethylamino)butyl]-1-piperidinyl]acetyl]-6H-pyrido-[2,3-b][1,4]benzodiazepin-6-on und 2,2-Dimethylbuttersäurechlorid in einer Ausbeute von 64 % der Theorie.
Farblose Kristalle vom Fp. 153-155°C (Diethylether).

Beispiel 19

5,11-Dihydro-11-[[4-[4-[(2,2-dimethyl-1-oxopentyl)ethylamino]butyl]-1-piperidinyl]acetyl]-6H-pyrido[2,3-b][1,4]-benzodiazepin-6-on

Hergestellt analog Beispiel 1 aus 5,11-Dihydro-11-[[4-[4-(ethylamino)butyl]-1-piperidinyl]acetyl]-6H-pyrido-[2,3-b][1,4]benzodiazepin-6-on und 2,2-Dimethyl-valeriansäurechlorid in einer Ausbeute von 69 % der Theorie.
Farblose Kristalle vom Fp. 168-169°C (Essigsäureethylester).

Beispiel 20

5,11-Dihydro-8-chlor-11-[[4-[4-[(2,2-dimethyl-1-oxopropyl)ethylamino]butyl]-1-piperidinyl]acetyl]-6H-pyrido-[2,3-b][1,4]benzodiazepin-6-on

Hergestellt analog Beispiel 1 aus 5,11-Dihydro-8-chlor-11-[[4-[4-(ethylamino)butyl]-1-piperidinyl]acetyl]-6H-pyrido-[2,3-b] [1,4] benzodiazepin-6-on und Pivalinsäurechlorid in einer Ausbeute von 55 % der Theorie.
Farblose Kristalle vom Fp. 205-207°C (Essigsäureethylester).

Beispiel 21

5,11-Dihydro-8-chlor-11-[[4- [4-[(2,2-dimethyl-1-oxopentyl)ethylamino]butyl]-1-piperidinyl]acetyl]-6H-pyrido-[2,3-b][1,4]benzodiazepin-6-on

Hergestellt analog Beispiel 1 aus 5,11-Dihydro-8-chlor-11-[[4-[4-(ethylamino)butyl]-1-piperidinyl]acetyl]-6H-pyrido-[2,3-b] [1,4]benzodiazepin-6-on und 2,2-Dimethylvaleriansäurechlorid in einer Ausbeute von 60 % der Theorie.
Farblose Kristalle vom Fp. 149-151°C (Essigsäureethylester).

Beispiel 22

5,11-Dihydro-8-chlor-11-[[4-[3-[(2,2-dimethyl-1-oxopentyl)-ethylamino]propyl]-1-piperidinyl]acetyl]-6H-pyrido[2,3-b]-[1,4]benzodiazepin-6-on

In einer Lösung von 0,52 g (4 mMol) 2,2-Dimethylvaleriansäure und 0,54 g (4 mMol) 1-Hydroxybenzotriazol in 50 ml Tetrahydrofuran werden bei 0°C unter Rühren 0,82 g (4 mMol) N.N'-Dicyclohexylcarbodiimid eingetragen. Man rührt 30 Minuten bei der gleichen Temperatur weiter und fügt dann 1,8 g (4 mMol) 5,11-Dihydro-8-chlor-11-[[4-[3-(ethylamino)propyl]-1-piperidinyl]acetyl]-6H-pyrido[2,3-b][1,4]benzodiazepin-6-on hinzu. Danach rührt man bei Zimmertemperatur 12 Stunden weiter. Man saugt vom gebildeten Dicyclohexylharnstoff ab und engt das Filtrat im Vakuum zur Trockene ein. Der Rückstand wird chromatographisch an Kieselgel (Firma Baker) mit einem Gemisch aus Essigsäureethylester/Methanol/Cyclohexan/Ammoniak = 8:1:1:0,1 als Elutionsmittel gereinigt. Man erhält farblose Kristalle vom Fp. 172-173°C (Essigsäureethylester).
Ausbeute: 160 mg = 7 % der Theorie.
Die Substanz ist entsprechend ihren physikalisch-chemischen und spektroskopischen Daten völlig identisch mit der nach Beispiel 12 erhaltenen Substanz.

Beispiel 23

5,11-Dihydro-11-[[4-[3-[(2,2-dimethyl-1-oxopentyl)amino]propyl]-1-piperidinyl]acetyl]-6H-pyrido[2,3-b][1,4]benzodiazepin-6-on

Herstellung analog Beispiel 1 aus 5,11-Dihydro-11-[[4-(3-aminopropyl)-1-piperidinyl]acetyl]-6H-pyrido[2,3-b][1,4]-benzodiazepin-6-on und 2,2-Dimethyl-valeriansäurechlorid in einer Ausbeute von 64 % der Theorie.
Farblose Kristalle vom Fp. 150-152°C (Essigsäureethylester).

Beispiel 24

5,10-Dihydro-5-[[4-[3-[(2,2-dimethyl-1-oxopentyl)ethylamino]propyl]-1-piperidinyl]acetyl]-11H-dibenzo[b,e][1,4]diazepin-11-on

Hergestellt analog Beispiel 1 aus 5,10-Dihydro-5-[[4-[3-ethylamino]propyl]-1-piperidinyl]acetyl]-11H-dibenzo-[b,e][1,4]diazepin-11-on.

Die Reinigung erfolgte durch Chromatographie an Kieselgel (Firma Merck, 30-60 µm) mit einer Mischung von Essigsäureethylester/Ammoniak (10:0,1) als Elutionsmittel.
Ausbeute: 53 % der Theorie.
Farblose Kristalle vom Fp. 124-126°C (Diethylether).
$R_F$-Wert im Dünnschichtchromatogramm: 0,5 (DC-Platten: Kieselgel, Firma Merck; Fließmittel: Methylenchlorid/Cyclohexan/Methanol/Ammoniak = 6,8:1,5:1,5:0,2.
Im folgenden wird die Herstellung pharmazeutischer Anwendungsformen anhand einiger Beispiele beschrieben:

Beispiel I

Tabletten mit 5 mg 5,11-Dihydro-11-[[4-[3-[(2,2-dimethyl)-1-oxo-butyl)ethyl-amino]propyl]-1-piperidinyl]acetyl]-6H-pyrido[2,3-b][1,4]benzodiazepin-6-on

| Zusammensetzung: | |
|---|---|
| 1 Tablette enthält: | |
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45°C getrocknet, nochmals durch obiges Sieb gerieben, mit Magnesiumstearat vermischt und zu Tabletten verpreßt.

| | |
|---|---|
| Tablettengewicht: | 220 mg |
| Stempel: | 9 mm |

Beispiel II

Dragées mit 5 mg 5,11-Dihydro-11-[[4-[3-[(2,2-dimethyl)-1-oxo-butyl)ethyl-amino]propyl]-1-piperidinyl]acetyl]-6H-pyrido[2,3-b] [1,4]benzodiazepin-6-on

Die nach Beispiel I hergestellten Tabletten werden nach bekanntem Verfahren mit einer Hülle überzogen, die im wesentlichen aus Zucker und Talkum besteht. Die fertigen Dragees werden mit Hilfe von Bienenwachs poliert.
Dragéegewicht: 300 mg

Beispiel III

Ampullen mit 10 mg 5,11-Dihydro-11-[[4-[4-[(2,2-dimethyl)-1-oxo-pentyl)ethyl-amino]butyl]-1-piperidinyl]acetyl]-6H-pyrido[2,3-b][1,4]benzodiazepin-6-on-dihydrochlorid

| Zusammensetzung: | |
|---|---|
| 1 Ampulle enthält: | |
| Wirkstoff | 10,0 mg |
| Natriumchlorid | 8,0 mg |
| Dest. | 1 ml |
| Wasser ad | |

Herstellungsverfahren:

Die Wirksubstanz und Natriumchlorid werden in dest. Wasser gelöst und anschließend auf das gegebene Volumen aufgefüllt. Die Lösung wird sterilfiltriert und in 1 ml-Ampullen abgefüllt.
Sterilisation: 20 Minuten bei 120°C.

Beispiel IV

Suppositorien mit 20 mg 5,11-Dihydro-8-chlor-11-[[4-[3-[(2,2-dimethyl-1-oxo-pentyl)ethyl-amino]propyl]-1-piperidinyl]acetyl]-6H-pyrido[2,3-b][1,4]benzodiazepin-6-on

| Zusammensetzung: | |
|---|---|
| 1 Zäpfchen enthält: | |
| Wirkstoff | 20,0 mg |
| Zäpfchenmasse (z.B. Witepsol W 45$^{(R)}$) | 1 680,0 mg |
| | 1 700,0 mg |

Herstellungsverfahren:

Die feinpulverisierte Wirksubstanz wird in der geschmolzenen und auf 40°C abgekühlten Zäpfchenmasse suspendiert. Man gießt die Masse bei 37°C in leicht vorgekühlte Zäpfchenformen aus.
Zäpfchengewicht 1,7 g

Beispiel V

Tropfen mit 5,11-Dihydro-11-[[4-[4-[(2,2-dimethyl)-1-oxo-pentyl)ethyl-amino]butyl]-1-piperidinyl]acetyl]-6H-pyrido [2,3-b][1,4]benzodiazepin-6-on-dihydrochlorid

Zusammensetzung:

100 ml Tropflösung enthalten:

| | | | |
|---|---|---|---|
| p-Hydroxybenzoesäuremethylester | | 0,035 | g |
| p-Hydroxybenzoesäurepropylester | | 0,015 | g |
| Anisöl | | 0,05 | g |
| Menthol | | 0,06 | g |
| Ethanol rein | | 10,0 | g |
| Wirkstoff | | 0,5 | g |
| Natriumcyclamat | | 1,0 | g |
| Glycerin | | 15,0 | g |
| Dest. Wasser | ad | 100,0 | ml |

Herstellungsverfahren:

Die Wirksubstanz und Natriumcyclamat werden in ca. 70 ml Wasser gelöst und Glycerin zugefügt. Man löst p-Hydroxybenzoesäureester, Anisöl sowie Menthol in Ethanol und fügt diese Lösung unter Rühren der wäßrigen Lösung zu. Abschließend wird mit Wasser auf 100 ml aufgefüllt und schwebeteilchenfrei filtriert.

**Patentansprüche**

**1.** Kondensierte Diazepinone der allgemeinen Formel I

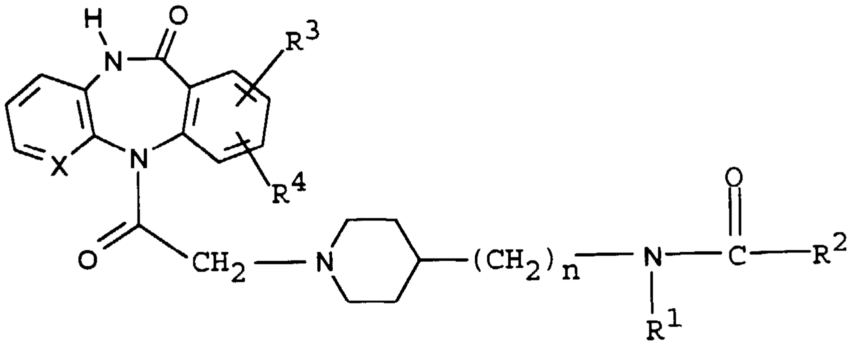

(I)

in der

X ein Stickstoffatom,

n die Zahlen 3 und 4,

$R^1$ eine geradkettige Alkylgruppe mit 1 bis 4 Kohlenstoffatomen,

$R^2$ eine verzweigte Alkylgruppe mit 4 bis 6 Kohlenstoffatomen, eine Cyclopropyl-, Cyclobutyl-, Cyclopentyl-, Cyclohexyl-, Cycloheptyl-, Methylcyclopentyl-, Methylcyclohexyl-, Methylcycloheptyl- oder Adamantylgruppe,

$R^3$ und $R^4$ Wasserstoffatome oder einer dieser Reste eine Methylgruppe oder ein Halogenatom bedeuten,

und die Verbindung, in der

X die =CH-Gruppe,

n die Zahl 3,

$R^1$ die Ethylgruppe,

$R^2$ die 1,1-Dimethylbutylgruppe,

$R^3$ und $R^4$ Wasserstoffatome bedeuten,

und ihre Salze mit anorganischen oder organischen Säuren.

**2.** Kondensierte Diazepinone der allgemeinen Formel I gemäß Anspruch 1, in denen $R^1$ eine Ethylgruppe darstellt, und ihre Salze mit anorganischen oder organischen Säuren.

**3.** Folgende Verbindungen der allgemeinen Formel I gemäß Anspruch I:

5,11-Dihydro-11-[[4-[3-[(2,2-dimethyl-1-oxobutyl]ethylamino]propyl]-1-piperidinyl]acetyl]-6H-pyrido[2,3-b][1,4]benzodiazepin-6-on,

5,11-Dihydro-11-[[4-[3-[(2,2-dimethyl-1-oxopentyl)ethylamino]propyl]-1-piperidinyl]acetyl]-6H-pyrido[2,3-b][1,4]benzodiazepin-6-on,

5,11-Dihydro-11-[[4-[4-[(2,2-dimethyl-1-oxopentyl)ethylamino]-butyl]-1-piperidinyl]acetyl]-6H-pyrido[2,3-b][1,4]benzodiazepin-6-on,

5,11-Dihydro-8-chlor-11-[[4-[3-[(2,2-dimethyl-1-oxopentyl)-ethylamino]propyl]-1-piperidinyl]acetyl]-6H-pyrido[2,3-b]-[1,4]benzodiazepin-6-on,

5,11-Dihydro-8-methyl-11-[[4-[3-[(2,2-dimethyl-1-oxopropyl)-ethylamino]propyl]-1-piperidinyl]acetyl]-6H-pyrido[2,3-b]-[1,4]benzodiazepin-6-on,

5,11-Dihydro-11-[[4-[3-[(2,2-dimethyl-1-oxopropyl)ethylamino]propyl]-1-piperidinyl]acetyl]-6H-pyrido[2,3-b][1,4]-benzodiazepin-6-on,

5,11-Dihydro-8-methyl-11-[[4-[3-[(2,2-dimethyl-1-oxobutyl)-ethylamino]propyl]-1-piperidinyl]acetyl]-6H-pyrido[2,3-b]-[1,4]benzodiazepin-6-on und

5,10-Dihydro-5-[[4-[3-[(2,2-dimethyl-1-oxopentyl)ethylamino]propyl]-1-piperidinyl]acetyl]-11H-dibenzo[b,e][1,4]diazepin-11-on

und ihre Salze mit anorganischen oder organischen Säuren.

**4.** Physiologisch verträgliche Salze mit anorganischen oder organischen Säuren der Verbindungen gemäß den Ansprüchen 1 bis 3.

**5.** Arzneimittelzubereitungen enthaltend mindestens ein kondensiertes Diazepinon gemäß Anspruch 1 bis 3 oder ein physiologisches verträgliches Salz gemäß Anspruch 4 neben üblichen Träger- und/oder Hilfsstoffen.

**6.** Verwendung von kondensierten Diazepinonen gemäß Anspruch 1 bis 4 zur Herstellung von Arzneimitteln zur Bekämpfung arteriosklerotisch bedingter Störungen der Cerebraldurchblutung bzw. zur Therapie von Erkrankungen des Zentralnervensystems.

**7.** Verwendung von kondensierten Diazepinonen gemäß den Ansprüchen 1 bis 4 und 6 zur Herstellung von Arzneimitteln zur Steigerung der Lernfähigkeit und Verbesserung des Gedächtnisses.

**8.** Verfahren zur Herstellung von kondensierten Diazepinonen der allgemeinen Formel I gemäß den Ansprüchen 1 bis 4, dadurch gekennzeichnet, daß

a) basisch substituierte, kondensierte Diazepinone der allgemeinen Formel II

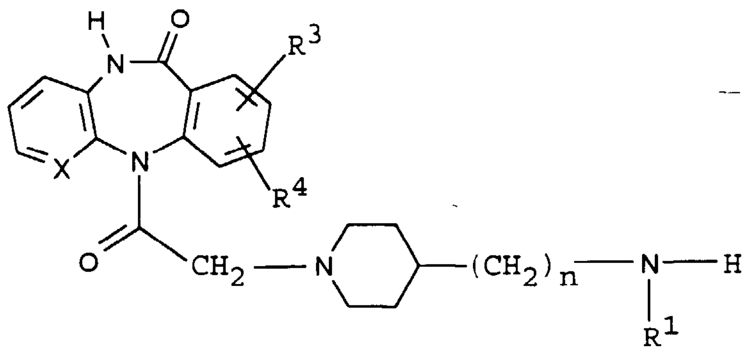

(II)

in der
X, n, $R^1$, $R^3$ und $R^4$ wie im Anspruch 1 definiert sind, mit Carbonsäuren der allgemeinen Formel III

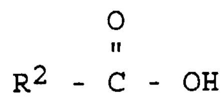

, (III)

in der
$R^2$ wie im Anspruch 1 definiert ist, oder mit deren reaktionsfähigen Derivaten in einem Lösungsmittel bei Temperaturen bis zum Siedepunkt des Reaktionsgemisches umgesetzt werden oder

b) Diazepinone der allgemeinen Formel IV

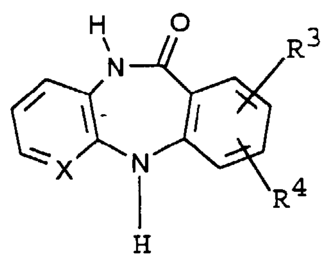

(IV),

worin
X, $R^3$ und $R^4$ wie im Anspruch 1 definiert sind, mit Carbonsäurederivaten der allgemeinen Formel V

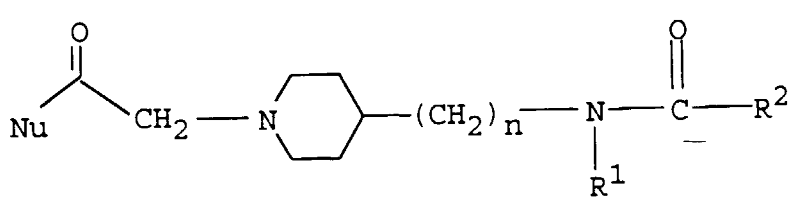

(V)

in der
n, $R^1$ und $R^2$ wie im Anspruch 1 definiert sind und Nu eine nucleofuge Gruppe darstellt, in einem Lösungsmittel umgesetzt werden

und, gewünschtenfalls, anschließend erhaltene Verbindungen der allgemeinen Formel I in ihre Diastereomeren bzw. enantiomeren Formen aufgetrennt und/oder die erhaltenen Salze in die freien Basen und/oder die erhaltenen freien Basen in ihre Säureadditionssalze, insbesondere in ihre physiologisch verträglichen Salze, mit anorganischen oder organischen Säuren übergeführt werden.

**Claims**

1. Condensed diazepinones of general formula I

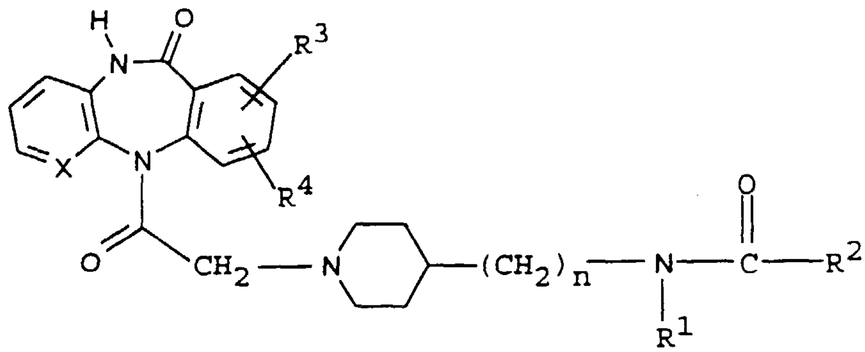

(I)

wherein

X is a nitrogen atom,

n represents the number 3 or 4,

$R^1$ is a straight-chained $C_{1-4}$alkyl group,

$R^2$ is a branched $C_{4-6}$alkyl group, a cyclopropyl, cyclobutyl, cyclopentyl, cyclohexyl, cycloheptyl, methylcyclopentyl, methylcyclohexyl, methylcycloheptyl or adamantyl group,

$R^3$ and $R^4$ represent hydrogen atoms or one of these groups represents a methyl group or a halogen atom,

and the compound wherein

X is the =CH group,

n denotes the number 3,

$R^1$ denotes the ethyl group,

$R^2$ denotes the 1,1-dimethylbutyl group, and

$R^3$ and $R^4$ denote hydrogen atoms,

and the salts thereof with inorganic or organic acids.

2. Condensed diazepinones of general formula I according to claim 1 wherein $R^1$ represents an ethyl group, and the salts thereof with inorganic or organic acids.

3. The following compounds of general formula I according to claim 1:

5,11-dihydro-11-[[4-[3-[(2,2-dimethyl-1-oxobutyl)-ethylamino]propyl]-1-piperidinyl]acetyl]-6H-pyrido [2,3-b] [1,4]benzodiazepin-6-one,

5,11-dihydro-[[4-[3-[(2,2-dimethyl-1-oxopentyl)ethylamino]propyl]-1-piperidinyl]acetyl]-6H-pyrido[2,3-b]-[1,4] benzodiazepin-6-one,

5,11-dihydro-11-[[4-[4-[(2,2-dimethyl-1-oxopentyl)-ethylamino]butyl]-1-piperidinyl]acetyl]-6H-pyrido[2,3-b] [1,4]benzodiazepin-6-one,

5,11-dihydro-8-chloro-11-[[4-[3-[(2,2-dimethyl-1-oxopentyl)ethylamino]propyl]-1-piperidinyl]acetyl]-6H-pyrido [2,3-b] [1,4]benzodiazepin-6-one,

5,11-dihydro-8-methyl-11-[[4-[3-[(2,2-dimethyl-1-oxopropyl)ethylamino]propyl]-1-piperidinyl]acetyl]-6H-pyrido[2,3-b][1,4]benzodiazepin-6-one,

5,11-dihydro-11-[[4-[3-[(2,2-dimethyl-1-oxopropyl)-ethylamino]propyl]-1-piperidinyl]acetyl]-6H-pyrido-[2,3-b][1,4]benzodiazepin-6-one and

5,11-dihydro-8-methyl-11-[[4-[3-[(2,2-dimethyl-1-oxobutyl)-ethylamino]propyl]-1-piperidinyl]acetyl]-6H-pyrido[2,3-b] [1,4]benzodiazepin-6-one and

5,10-dihydro-5-[[4-[3-[(2,2-dimethyl-1-oxopentyl)-ethylamino]-propyl]-1-piperidinyl]acetyl]-11H-dibenzo[b,e][1,4]diazepin-11-one

and the salts thereof with inorganic or organic acids.

**4.** Physiologically acceptable salts with inorganic or organic acids of the compounds according to claims 1 to 3.

**5.** Pharmaceutical preparations containing at least one condensed diazepinone according to claims 1 to 3 or a physiologically acceptable salt according to claim 4 together with conventional carriers and/or excipients.

**6.** Use of condensed diazepinones according to claims 1 to 4 for preparing pharmaceutical compositions for the treatment of arteriosclerotically induced disorders of cerebral blood flow or for the treatment of diseases of the central nervous system.

**7.** Use of condensed diazepinones according to claims 1 to 4 and 6 for preparing pharmaceutical compositions for increasing learning capacity and for improving memory.

**8.** Process for preparing condensed diazepinones of general formula I according to claims 1 to 4, characterised in that

a) base-substituted condensed diazepinones of general formula II

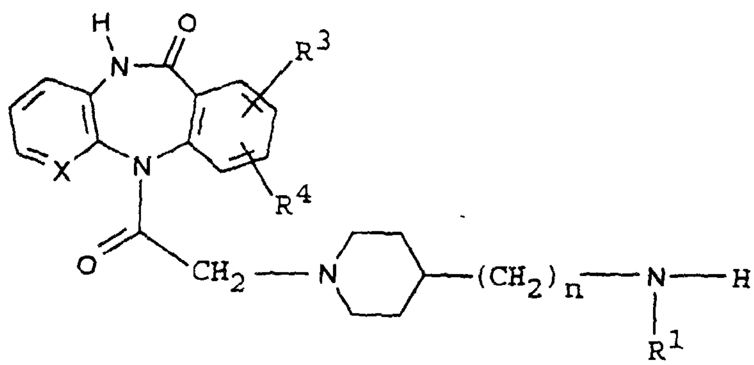

(II)

wherein
X, n, $R^1$, $R^3$ and $R^4$ are defined as in claim 1, are reacted with carboxylic acids of general formula III

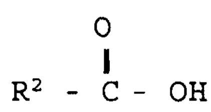

(III

wherein
$R^2$ is defined as in claim 1, or with the reactive derivatives thereof in a solvent at temperatures up to the boiling point of the reaction mixture, or

b) diazepinones of general formula IV

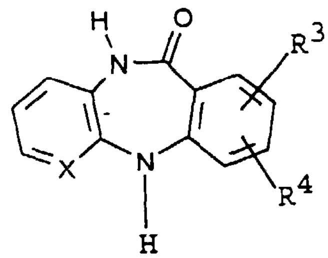

(IV),

wherein
X, $R^3$ and $R^4$ are defined as in claim 1, are reacted with carboxylic acid derivatives of general formula V

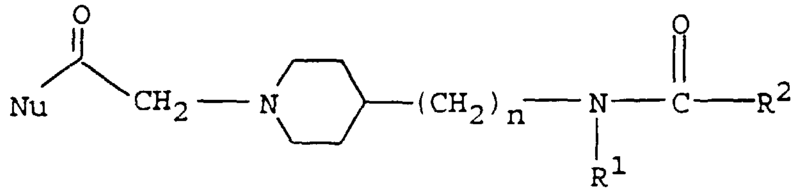

(V)

wherein
n, $R^1$ and $R^2$ are defined as in claim 1 and
Nu represents a nucleofugic group, in a solvent, and
subsequently, if desired, compounds of general formula I obtained are separated into their diastereomers or enantiomeric forms and/or salts obtained are converted into the free bases and/or free bases obtained are converted into the acid addition salts thereof, more particularly the physiologically acceptable salts thereof, with inorganic or organic acids.

**Revendications**

**1.** Diazépinones condensées de formule générale I

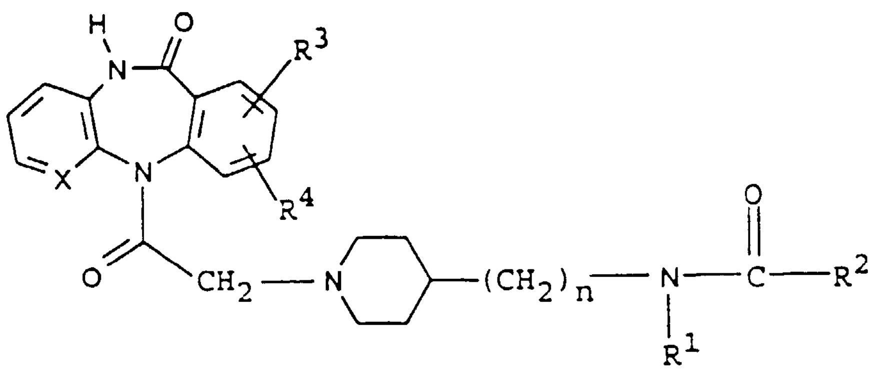

(I)

dans laquelle

X représente un atome d'azote,
n représente les nombres 3 et 4,
$R^1$ représente un groupe alkyle linéaire de 1 à 4 atomes de carbone,
$R^2$ représente un groupe alkyle ramifié de 4 à 6 atomes de carbone, un groupe cyclopropyle, cyclobutyle, cyclopentyle, cyclohexyle, cycloheptyle, méthylcyclopentyle, méthylcyclohexyle, méthylcycloheptyle ou adamantyle,
$R^3$ et $R^4$ représentent des atomes d'hydrogène ou l'un de ces restes représente un groupe méthyle ou un atome d'halogène,

et le composé dans lequel

X représente le groupe =CH-,

n représente le nombre 3,
$R^1$ représente le groupe éthyle,
$R^2$ représente le groupe 1,1-diméthylbutyle,
$R^3$ et $R^4$ représentent des atomes d'hydrogène,

et leurs sels avec des acides inorganiques ou organiques.

**2.** Diazépinones condensées de formule générale I selon la revendication 1, dans lesquelles $R^1$ représente un groupe éthyle, et leurs sels avec des acides inorganiques ou organiques.

**3.** Composés suivants de formule générale I selon la revendication 1:

5,11-dihydro-11-[[4-[3-[(2,2-diméthyl-1-oxobutyl)éthylamino]propyl]-1-pipéridinyl]acétyl]-6H-pyrido[2,3-b][1,4]-benzodiazépin-6-one,
5,11-dihydro-11-[[4-[3-[(2,2-diméthyl-1-oxopentyl)éthylamino]propyl]-1-pipéridinyl]acétyl]-6H-pyrido[2,3-b][1,4]-benzodiazépin-6-one,
5,11-dihydro-11-[[4-[4-[(2,2-diméthyl-1-oxopentyl)éthylamino]butyl]-1-pipéridinyl]acétyl]-6H-pyrido[2,3-b][1,4]-benzodiazépin-6-one,
5,11-dihydro-8-chloro-11-[[4-[3-[(2,2-diméthyl-1-oxopentyl)-éthylamino]propyl]-1-pipéridinyl]acétyl]-6H-pyrido[2,3-b]-[1,4]benzodiazépin-6-one,
5,11-dihydro-8-méthyl-11-[[4-[3-[(2,2-diméthyl-1-oxopropyl)-éthylamino]propyl]-1-pipéridinyl]acétyl]-6H-pyrido[2,3-b][1,4]benzodiazépin-6-one,
5,11-dihydro-11-[[4-[3-[(2,2-diméthyl-1-oxopropyl)éthylamino]propyl]-1-pipéridinyl]acétyl]-6H-pyrido[2,3-b][1,4]-benzodiazépin-6-one,
5,11-dihydro-8-méthyl-11-[[4-[3-[(2,2-diméthyl-1-oxobutyl)-éthylamino]propyl]-1-pipéridinyl]acétyl]-6H-pyrido[2,3-b][1,4]benzodiazépin-6-one et
5,10-dihydro-5-[[4-[3-[(2,2-diméthyl-1-oxopentyl)éthylamino]propyl]-1-pipéridinyl]acétyl]-11H-dibenzo[b,e][1,4]-diazépin-6-one

et leurs sels avec des acides inorganiques ou organiques.

**4.** Sels physiologiquement acceptables avec des acides inorganiques ou organiques des composés selon les revendications 1 à 3.

**5.** Préparations médicamenteuses contenant au moins une diazépinone condensée selon les revendications 1 à 3 ou un sel physiologiquement acceptable selon la revendication 4 outre des supports et/ou adjuvants habituels.

**6.** Utilisation de diazépinones condensées selon les revendications 1 à 4 pour la préparation de médicaments pour la lutte contre les troubles de l'irrigation cérébrale d'origine artériosclérеuse ou pour la thérapie de maladies du système nerveux central.

**7.** Utilisation de diazépinones condensées selon les revendications 1 à 4 et 6 pour la préparation de médicaments pour augmenter la capacité d'apprentissage et pour améliorer la mémoire.

**8.** Procédé de préparation de diazépinones condensées de formule générale I selon les revendications 1 à 4, caractérisé en ce que

a) des diazépinones condensées substituées de manière basique de formule générale II

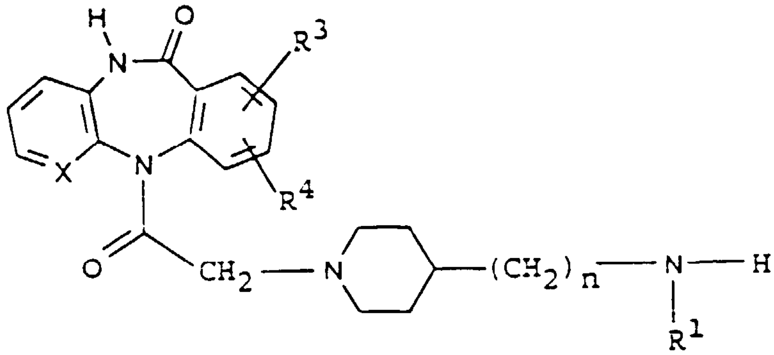

(II)

dans laquelle
X, n, $R^1$, $R^3$ et $R^4$ sont définis comme dans la revendication 1, sont mises à réagir avec des acides carboxyliques de formule générale III

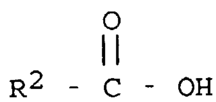

(III)

dans laquelle
$R^2$ est défini comme dans la revendication 1, ou avec leurs dérivés réactifs dans un solvant à des températures pouvant atteindre le point d'ébullition du mélange réactionnel ou
b) des diazépinones de formule générale IV

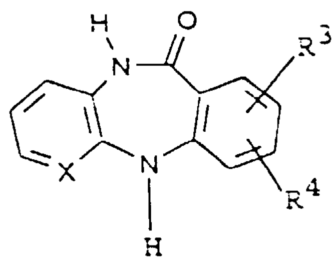

(IV)

dans laquelle
X, $R^3$ et $R^4$ sont définis comme dans la revendication 1, sont mises à réagir dans un solvant avec des dérivés d'acides carboxyliques de formule générale V

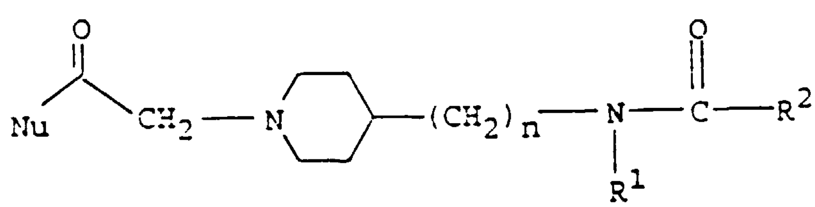

(V)

dans laquelle
n, $R^1$ et $R^2$ sont définis comme dans la revendication 1 et Nu représente un groupe nucléofuge
et, si on le souhaite, les composés de formule générale I obtenus ensuite sont résolus en leurs diastéréoisomères ou en leurs formes énantiomères et/ou les sels obtenus sont convertis en les bases libres et/ou les bases libres obtenues sont converties en leurs sels d'addition d'acide, en particulier en leurs sels physiologiquement acceptables, avec des acides inorganiques ou organiques.

Figur 1

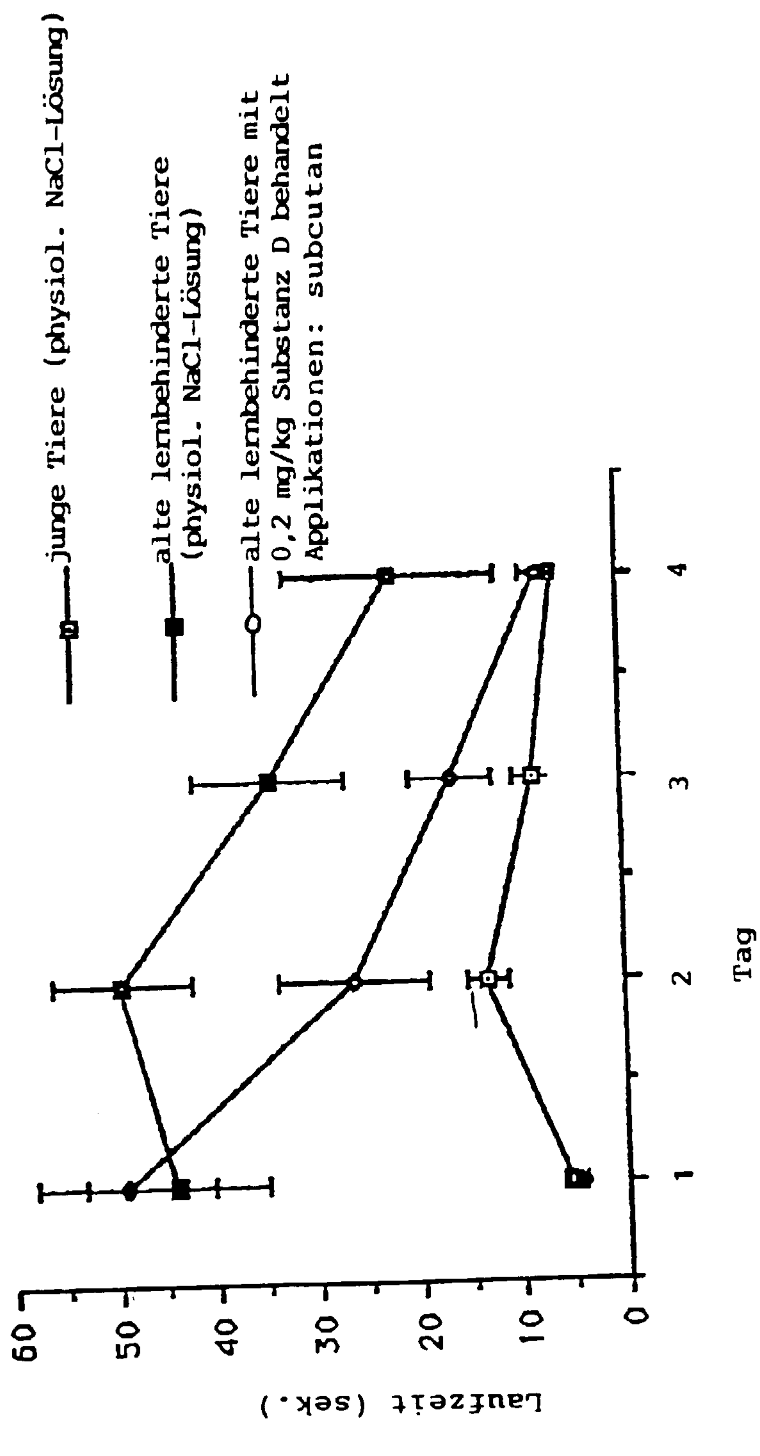
junge Tiere (physiol. NaCl-Lösung)
alte lernbehinderte Tiere (physiol. NaCl-Lösung)
alte lernbehinderte Tiere mit 0,2 mg/kg Substanz D behandelt
Applikationen: subcutan
Laufzeit (sek.)
0
10
20
30
40
50
60
Tag
1
2
3
4